# EUROPÄISCHE PATENTSCHRIFT

(11) **EP 1 740 948 B1**
(45) Veröffentlichungstag und Bekanntmachung des Hinweises auf die Patenterteilung: **12.09.2007**
(21) Anmeldenummer: 05715799.2
(22) Anmeldetag: 07.03.2005
(51) Int. Cl.: G01N 33/543, B01L 3/00, G02B 1/10, G02B 6/12, H01L 21/762, H01L 21/306

(54) **PARTIELL OXIDIERTES MAKROPORÖSES SILIZIUM MIT NICHTZUSAMMENHÄNGENDEN SILIZIUMWÄNDEN**
PARTIALLY OXIDISED MACROPOROUS SILICON COMPRISING DISCONTINUOUS SILICON WALLS
SILICIUM MACROPOREUX PARTIELLEMENT OXYDE COMPORTANT DES PAROIS DE SILICIUM DISCONTINUES

(30) Priorität: 19.04.2004 DE 102004018846
(43) Veröffentlichungstag der Anmeldung: 10.01.2007
(73) Patentinhaber: Qimonda AG, 81739 München (DE)
(72) Erfinder: DERTINGER, Stephan, 80335 München (DE); KLÜHR, Marco, 81673 München (DE)
(74) Vertreter: Müller-Boré & Partner Patentanwälte
(86) Internationale Anmeldenummer: PCT/EP2005/002390
(87) Internationale Veröffentlichungsnummer: WO 2005/100994

(56) Entgegenhaltungen:
- WO-A-01/24929
- WO-A-03/089925
- WO-A-03/089931
- US-A1- 2002 191 884
- US-A1- 2004 045 932
- MASINI G ET AL: "Si based optoelectronics for communications" MATERIALS SCIENCE AND ENGINEERING B, ELSEVIER SEQUOIA, LAUSANNE, CH, Bd. 89, Nr. 1-3, 14. Februar 2002 (2002-02-14), Seiten 2-9, XP004334357 ISSN: 0921-5107
- BIRNER A ET AL: "SILICON-BASED PHOTONIC CRYSTALS" ADVANCED MATERIALS, VCH VERLAGSGESELLSCHAFT, WEINHEIM, DE, Bd. 13, Nr. 6, 16. März 2001 (2001-03-16), Seiten 377-388, XP001039026 ISSN: 0935-9648

## Beschreibung

Die vorliegende Erfindung betrifft eine Vorrichtung, umfassend ein flächig ausgebildetes makroporöses Trägermaterial auf Silizium-Basis, das über mindestens einen Oberflächenbereich verteilt eine Vielzahl von Poren mit einem Durchmesser im Bereich von 500 nm bis 100 µm aufweist, welche sich von einer Oberfläche (10A) zur gegenüberliegenden Oberfläche (10B) des Trägermaterials durchgängig erstrecken, wobei die Vorrichtung zwei oder mehrere Bereiche (11A) aufweist, die jeweils zwei oder mehrere Poren mit Porenwänden aus SiO₂ umfassen, und wobei diese Bereiche jeweils von einem zu den Längsachsen der Poren im wesentlichen parallel angeordneten, zu den Oberflächen (10A, 10B) hin offenen Rahmen bzw. Kasten (12) aus Wänden mit einem Siliziumkern (12A) umgeben ist, wobei der Kern aus Silizium über den Querschnitt zur Außenseite der den Rahmen bildenden Wände hin in Siliziumdioxid übergeht, und wobei jeder einzelne Rahmen (12) innerhalb der Gesamtheit aller Rahmen aus Wänden mit einem Siliziumkern (12A) vollständig von den ihn umgebenden bzw. benachbarten Rahmen räumlich isoliert ist, sowie ein Verfahren zu deren Herstellung. Die erfindungsgemäße Vorrichtung eignet sich als Basis für ein "BioChip-Grundmodul" in Verfahren zum Nachweis biochemischer (Bindungs)Reaktionen sowie hierfür insbesondere zur Untersuchung von enzymatischen Reaktionen, Nukleinsäure-Hybridisierungen, Protein-Protein-Wechselwirkungen und anderer Bindungsreaktionen im Bereich der Genom-, Proteom-oder Wirkstoff-Forschung in Biologie und Medizin.

In der Molekularbiologie finden heute in zunehmendem Maße Biochips Verwendung, mit denen auf schnelle Art und Weise Erkenntnisse über Organismen und Gewebe gewonnen werden. Für die Biowissenschaften und die medizinische Diagnostik ist die Detektion (bio)chemischer Reaktionen, d.h. die Detektion biologisch relevanter Moleküle in definiertem Untersuchungsmaterial von herausragender Bedeutung. In diesem Rahmen wird die Entwicklung von sogenannten BioChips stetig vorangetrieben. Bei derartigen BioChips handelt es sich üblicherweise um miniaturisierte hybride Funktionselemente mit biologischen und technischen Komponenten, insbesondere auf einer Oberfläche eines BioChip-Grundmoduls immobilisierten Biomolekülen, die als spezifische Interaktionspartner dienen. Häufig weist die Struktur dieser Funktionselemente Reihen und Spalten auf. Man spricht dann von sogenannten "Mikroarrays". Da tausende von biologischen bzw. biochemischen Funktionselementen auf einem Chip angeordnet sein können, werden diese in der Regel mit mikrotechnischen Methoden angefertigt.

Als biologische und biochemische Funktionselemente kommen insbesondere DNA, RNA, PNA, (bei Nukleinsäuren und ihren chemischen Derivaten können z.B. Einzelstränge wie Oligonukleotide, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), einzelne Zellen, mehrzellige Organismen sowie Zellverbände in Frage.

Die am weitesten verbreitete Variante von Biochips sind die sogenannten Microarrays. Dies sind kleine Plättchen ("Chips") aus beispielsweise Glas, Gold, Kunstoff oder Silizium. Zum Nachweis entsprechender biologischer oder biochemischer (Bindungs)Reaktionen werden beispielsweise kleine Mengen an solubilisierten unterschiedlichen Fängermolekülen, z.B. eine bekannte Nukleinsäuresequenz, in Form von kleinsten Tröpfchen punktförmig und matrizenartig, sogenannte Dots, auf der Oberfläche des BioChip-Grundmoduls fixiert.

In der Praxis werden einige hundert bis einige tausend Tröpfchen pro Chip verwendet. Anschließend wird ein zu untersuchender Analyt, der beispielsweise fluoreszenzmarkierte Zielmoleküle enthalten kann, über diese Oberfläche gepumpt. Dabei kommt es im allgemeinen zu unterschiedlichen chemischen (Bindungs)Reaktionen zwischen den im Analyt enthaltenen Zielmolekülen und den fixierten bzw. immobilisierten Fängermolekülen. Wie bereits angeführt, werden zur Beobachtung dieser Reaktionen oder Bindungen die Zielmoleküle mit Farbstoffmolekülbausteinen, üblicherweise Fluorochromen markiert. Das Vorhandensein und die Intensität von Licht, das von den Fluorochromen emittiert wird, gibt Aufschluß über den Verlauf der Reaktion oder Bindung in den einzelnen Tröpfchen auf dem Substrat, so daß Rückschlüsse auf das Vorhandensein und/oder die Eigenschaft der Zielmoleküle und/oder Fängermoleküle gezogen werden können. Wenn sich die entsprechenden fluoreszenzmarkierten Zielmoleküle des Analyten mit bzw. an den an der Oberfläche des Trägersubstrats immobilisierten Fängermolekülen umsetzen bzw. binden, kann durch optische Anregung mit einem Laser und Messung des entsprechenden Fluoreszenzsignals diese Reaktion bzw. Bindung nachgewiesen werden.

Substrate mit hoher, aber definierter Porosität weisen als Basis für derartige BioChips mehrere Vorteile gegenüber planaren Substraten auf. Auf der stark vergrößerten Oberfläche können mehr Nachweisreaktionen stattfinden. Dadurch steigt die Nachweisempfindlichkeit für biologische Assays. Durch Pumpen der im Analyt gelösten Zielmoleküle durch die Kanäle zwischen Vorder- und Rückseite des porösen Substrates werden diese in nahen räumlichen Kontakt mit der Oberfläche des Substrates gebracht (< 10 µm). Auf dieser Größenskala ist die Diffusion ein sehr effektiver Transportprozess, der innerhalb kurzer Zeit die Distanzen zwischen nachzuweisendem Zielmolekül und dem auf der Oberfläche immobilisierten Fängermolekül überbrückt. Die Geschwindigkeit der Bindungsreaktion kann dadurch erhöht und damit die Dauer des Nachweisverfahrens deutlich verkürzt werden.

Ein Beispiel für ein Substrat mit derartiger definierter Porösität ist elektrochemisch hergestelltes poröses Silizium (vgl. DE 42 02 454, EP 0 553 465 oder DE 198 20 756).

Ein großer Teil der heute verwendeten analytischen Methoden in der Wirkstoff-Forschung und klinischen Diagnostik setzt optische Verfahren zum Nachweis von Bindungsereignissen zwischen nachzuweisender Substanz und Fängermolekülen ein (z.B. DNA-Hybridisierungen, Antikörper-Antigen-Wechselwirkungen und Protein-Wechselwirkungen). Die nachzuweisende Substanz wird hierbei mit einem Marker versehen, der nach Anregung mit Licht geeigneter Wellenlänge fluoresziert (Fluoreszenzverfahren) oder der eine chemische Reaktion auslöst, die wiederum Licht erzeugt (Chemilumineszenzverfahren). Bindet die nachzuweisende Substanz, d.h. das Zielmolekül, mit dem immobilisierten Fängermolekül auf der Oberfläche, so kann dies optisch, z.B. über Lumineszenz, nachgewiesen werden. Unter dem Begriff "Lumineszenz" wird hierbei die spontane Emission von Photonen im ultravioletten bis infraroten Spektralbereich bezeichnet. Anregungsmechanismen der Lumineszenz können optischer oder nicht-optischer Natur sein, beispielsweise elektrische, chemische, biochemische und/oder thermische Anregungsprozesse. Somit sollen insbesondere Chemi-, Bio- und Elektrolumineszenz sowie Fluoreszenz und Phosphoreszenz unter den Begriff "Lumineszenz" im Sinne dieser Erfindung fallen.

Poröse Substrate mit hoher optischer Dichte und geringer Reflektivität, wie beispielsweise poröses Silizium, dessen Reflektivität im sichtbaren Bereich des Spektrums 50 bis 70 % beträgt, liefern in Verbindung mit Fluoreszenz- oder Chemilumineszenzverfahren jedoch nicht die erwarteten Ergebnisse, insofern die experimentell beobachtete Lichtsignalausbeute bei weitem nicht an die theoretisch erreichbaren Werte heranreicht. Der Grund für die gegenüber den theoretischen Werten verringerte, experimentell beobachtete Lichtsignalausbeute beim Einsatz derartiger poröser Substrate liegt zum einen in Problemen bei der Emission der Lumineszenz der zu untersuchenden Substanz bzw. Bindung und zum anderen - wenn ein Fluoreszenzverfahren vorliegt - in Problemen bei der optischen Anregung der Fluoreszenz begründet.

Wird im gesamten Volumen der Poren (Luminenszenz-)Licht erzeugt, spielt die Reflektivität der Porenwände eine entscheidende Rolle für die effektive Herausleitung des optischen Signals zur Oberfläche. Im Falle der Chemilumineszenz wird das Lichtsignal isotrop in alle Raumrichtungen abgestrahlt. Infolgedessen strahlt nur ein sehr geringer Anteil des erzeugten Lichts direkt im Öffnungswinkel der einzelnen Pore ab. Alle anderen Strahlengänge werden an den Wänden der Poren mehrfach reflektiert, bis sie die Öffnung der jeweiligen Pore erreichen. Schon bei Reflektivitäten, die nur wenig unterhalb von 100 % liegen, ist jedoch die Intensität eines Signals nach mehreren Reflexionen stark vermindert. Dieser Effekt hat zur Folge, daß dieser Anteil des erzeugten Signals auf dem Weg aus der Pore stark abgeschwächt wird und kaum noch zum Gesamtsignal beitragen kann.

Ferner stellt auch die Abschwächung durch Vielfachreflektionen an den Porenwänden bei der Anregung und Emission der Fluoreszenz ein ernstzunehmendes Problem dar. Nur Fluorophore (fluoreszierende Substanzen im Analyten), welche direkt in Richtung der Porenöffnung abstrahlen, stehen unabgeschwächt für ein Fluoreszenzsignal zur Verfügung. Alle übrigen Strahlengänge werden an den Wänden der Poren mindestens einmal reflektiert bis sie die Öffnung der Pore erreichen. Schon bei Reflektivitäten, welche nur wenig unterhalb von 100% liegen, führen diese Vielfachreflektionen zu einer empfindlichen Abschwächung des zu detektierenden optischen Signals.

Um die oben genannten Probleme der Intensitätsabschwächung durch Vielfachreflektionen zu lösen, wurde vorgeschlagen, Reflexschichten zu einer Verringerung der Reflektionsverluste an den Porenwänden anzuordnen, um dadurch das Anregungs- und Emissionslicht besser aus der Poren herausleiten zu können. Jedoch konnte dieser Lösungsansatz keine signifikante Verbesserung der Signalausbeute bewirken.

In WO03/08992 wird eine 3D-Wellenleiterstruktur vorgestellt, welche lokal transparente Bereiche aus SiO₂ aufweist, wobei diese transparenten Bereiche wiederum von einem reflektierenden Rahmen aus Wänden mit Siliziumkern umgeben sind, wodurch das optische Übersprechen des in der Struktur erzeugten Lumineszenzlichts zwischen den Kompartments durch die reflektierenden Wände aus im wesentlichen Silizium unterbunden werden kann. Die in WO03/089925 vorgestellte Vorrichtung zeigt im Rahmen von Analyseverfahren auf Fluoreszenz- oder Chemilumineszenz-Basis Chemilumineszenz-Basis zwar eine verbesserte absolute Signalausbeute bei verbessertem Signal-Rausch-Verhältnis. Jedoch ist die Herstellung der in WO03/089925 vorgestellten 3D-Wellenleiterstruktur nur schwer zu kontrollieren, da während der thermischen Oxidation des makroporösen Siliziums durch die Volumenverdopplung beim Übergang von Silizium zu Siliziumdioxid starke Spannungen innerhalb der Struktur entstehen. Im ungünstigsten Fall führt dies zu einer Verbiegung und Verzerrung der Struktur. Für Biochips sind solche nicht-planaren Substrate aber ungeeignet.

Der vorliegenden Erfindung liegt somit die Aufgabe zugrunde, eine Vorrichtung bzw. ein "BioChip-Grundmodul" zum Nachweis biochemischer Reaktionen und/oder Bindungen bereitzustellen, die bzw. das im Rahmen von Analyseverfahren auf Fluoreszenz-oder Chemilumineszenz-Basis eine im Vergleich zu der in WO03/089925 vorgestellten Vorrichtung vergleichbar günstige absolute Signalausbeute bei vergleichbarem Signal-Rausch-Verhältnis liefern soll, um so die Nachweisempfindlichkeit von mit dem fertigen Biochip durchzuführenden Tests zu steigern, deren bzw. dessen Herstellung jedoch nicht die vorstehend angeführten Probleme der in WO03/089925 vorgestellten Vorrichtung zeigt.

Diese Aufgabe wird durch die in den Ansprüchen gekennzeichneten Ausführungsformen gelöst.

Insbesondere wird eine Vorrichtung bereitgestellt, umfassend ein flächig ausgebildetes makroporöses Trägermaterial (10) auf Silizium-Basis, das über mindestens einen Oberflächenbereich verteilt eine Vielzahl von periodisch angeordneten, diskreten Poren (11) mit einem Durchmesser im Bereich von 500 nm bis 100 µm aufweist, welche sich von einer Oberfläche (10A) zur gegenüberliegenden Oberfläche (10B) des Trägermaterials durchgängig erstrecken, wobei die Vorrichtung zwei oder mehrere Bereiche (11A) aufweist, die jeweils zwei oder mehrere Poren mit Porenwänden aus SiO₂ umfassen, und wobei diese Bereiche jeweils von einem zu den Längsachsen der Poren im wesentlichen parallel angeordneten, zu den Oberflächen (10A, 10B) hin offenen Rahmen bzw. Kasten (12) aus Wänden mit einem Siliziumkern (12A) umgeben ist, wobei der Kern aus Silizium über den Querschnitt zur Außenseite der den Rahmen bildenden Wände hin in Siliziumdioxid übergeht, und wobei jeder einzelne Rahmen (12) innerhalb der Gesamtheit aller Rahmen aus Wänden mit einem Siliziumkern (12A) vollständig von den ihn umgebenden bzw. benachbarten Rahmen räumlich isoliert ist.

Die erfindungsgemäße Vorrichtung weist lokal vollständig durchoxidierte Bereiche aus SiO₂ auf, d.h. Bereiche, die mehrere Poren mit Porenwänden aus SiO₂ umfassen. Diese durchoxidierten Bereiche sind wiederum von einer kastenartigen bzw. rahmenartigen Überstruktur umgeben. Die durchoxidierten Bereiche sind von Wänden aus im wesentlichen Silizium eingefasst bzw. umgeben, so daß diese Wände aus im wesentlichen Silizium einen zu den Oberflächen (10A, 10B) hin offenen Rahmen bzw. Kasten bzw. Zylinder, dessen Zylinderachse parallel zu den Poren verläuft, bilden, der die lokal vollständig durchoxidierten Bereiche aus SiO₂ umgibt bzw. umschließt. Die den Rahmen bildenden Wände weisen einen Kern aus Silizium auf, wobei, über einen in der Flächenebene des Trägermaterials verlaufenden Querschnitt gesehen, das Silizium zur Außenseite der Wände hin in Siliziumdioxid übergeht.

Gemäß der vorliegenden Erfindung ist jeder einzelne Rahmen (12) innerhalb der Gesamtheit aller Rahmen aus Wänden mit einem Siliziumkern (12A) vollständig von den ihn umgebenden Rahmen bzw. den benachbarten Rahmen räumlich isoliert. Die Siliziumwände der einzelnen Bereiche bzw. Kompartments hängen somit nicht zusammen bzw. berühren sich nicht, sondern sind über Bereiche mit Porenwänden aus SiO₂ voneinander vollständig getrennt. Aufgrund dieser erfindungsgemäßen strukturellen Anordnung wird eine räumliche Entkopplung der im Zuge der Herstellung einer solchen Vorrichtung durch die Volumenverdopplung beim Übergang von Silizium zu Siliziumdioxid lokal entstehenden Spannungen erreicht. In den durchoxidierten Bereichen sind die Wände zwischen den Poren vollständig aus SiO₂ aufgebaut. Diese Bereiche bzw. Kompartments sind damit für Wellenlängen insbesondere im sichtbaren Bereich transparent. Die erfindungsgemäße Vorrichtung weist somit lokal transparente Bereiche aus SiO₂ auf, wobei diese transparenten Bereiche wiederum von einem reflektierenden Rahmen aus Wänden mit Siliziumkern umgeben sind. Mit anderen Worten, es liegen lokal vollständig transparente Bereiche bzw. Kompartments aus SiO₂ vor, die durch nicht-transparente Wände mit Siliziumkern, welche in der erfindungsgemäßen Vorrichtung quasi eine diese Kompartments vollständig umgebende, kastenartige Sekundärstruktur bilden, voneinander.getrennt sind.

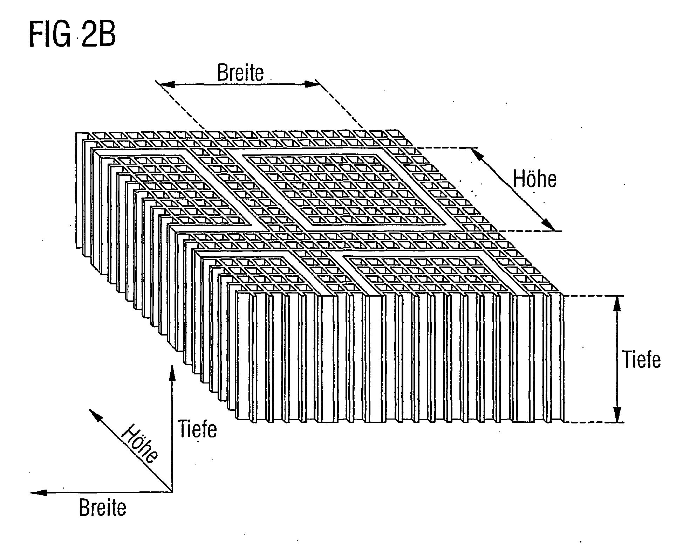

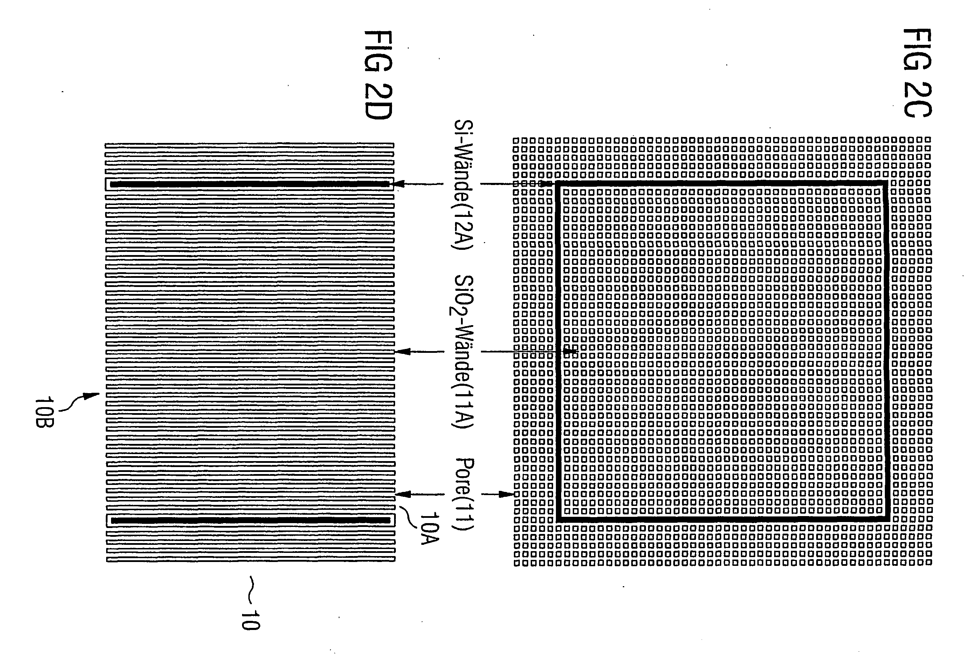

Im Rahmen der vorliegenden Erfindung kann der Siliziumrahmen der Kompartments ein Aspektverhältnis von etwa 1:1:100 bis 1:1:1 (Höhe x Breite x Tiefe) aufweisen; siehe Fig. 2 (B).

Der Rahmen bzw. Kasten (12) aus Wänden mit einem Siliziumkern, der zu den beiden Außenseiten hin jeweils in Siliziumdioxid übergeht, eliminiert Streulicht und optisches Übersprechen zwischen den Bereichen bzw. Kompartments, die zwei oder mehrere Poren mit Porenwänden aus SiO₂ umfassen. Dies ist ein wesentlicher Vorteil gegenüber porösen Substraten, die vollständig transparent sind (z.B. SiO₂, Glaschips oder Al₂O₃).

In der erfindungsgemäßen Vorrichtung ist über mindestens einen Oberflächenbereich des flächig ausgebildeten makroporösen Trägermaterials (10) verteilt eine Vielzahl von üblicherweise periodisch angeordneten Poren, welche sich von einer Oberfläche (10A) zur gegenüberliegenden Oberfläche (10B) des Trägermaterials erstrecken, angeordnet. Im Rahmen der vorliegenden Erfindung können auf dem flächig ausgebildeten makroporösen Trägermaterial (10) bereichsweise auch Sacklöcher, d.h. nur nach einer der Oberflächenseiten (10A, 10B) geöffnete Poren, vorgesehen werden.

Das eingesetzte makroporöse Trägermaterial weist üblicherweise einen Porendurchmesser von 1 µm bis 99 µm, vorzugsweise 1 bis 11 µm auf. Die Dicke des makroporösen Trägermaterials beträgt üblicherweise 100 bis 1.000 µm, vorzugsweise 250 bis 450 µm. Der Abstand von Porenmitte zu Porenmitte (Pitch), d.h. zweier zueinander benachbarter bzw. angrenzender Poren beträgt üblicherweise 1 bis 100 µm, vorzugsweise 2 bis 12 µm. Die Porendichte liegt üblicherweise im Bereich von 10⁴ bis 10⁸/cm².

Die Poren (11) in der erfindungsgemäßen Vorrichtung können beispielsweise im wesentlichen rund oder ellipsenförmig gestaltet sein. In einer bevorzugten Ausführungsform der vorliegenden Erfindung sind die Poren (11) mit Porenwänden aus SiO₂ im wesentlichen quadratisch ausgebildet. Der Rahmen (12) aus Wänden mit einem Siliziumkern (12A) kann dann in im wesentlichen quadratischer oder rechteckiger Form vorliegen.

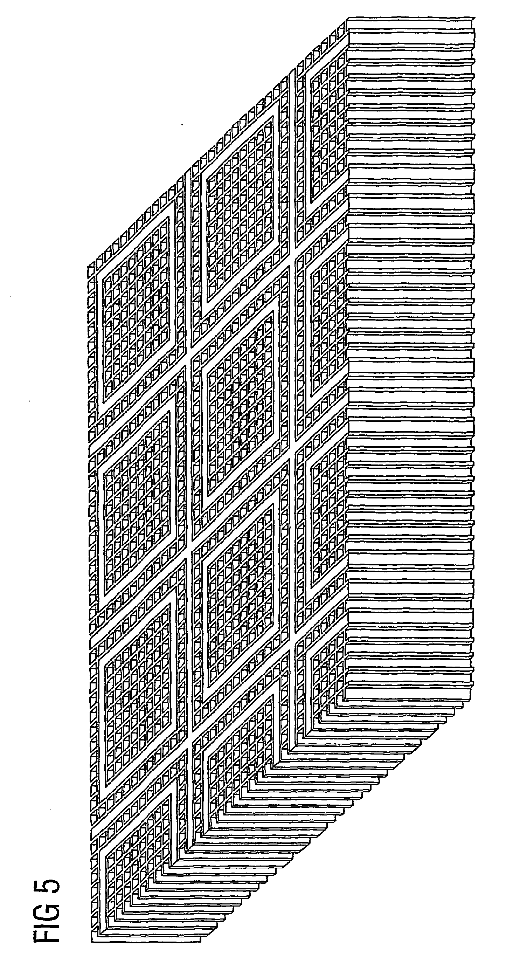

Im Rahmen der vorliegenden Erfindung ist auch eine Vorrichtung mit sogenannter Hybridstruktur eingeschlossen, die aus zusammenhängenden Kompartments und nichtzusammenhängenden Kompartments aufgebaut ist (siehe Fig. 5).

Die erfindungsgemäße Vorrichtung kann durch ein Verfahren bereitgestellt werden, umfassend die Schritte:
(a) Bereitstellen eines Trägermaterials aus Silizium mit den Oberflächen (10A, 10B);
(b) Erzeugen von Sacklöchern, deren Tiefe geringer als die Dicke des Trägermaterials ist, durch elektrochemisches Ätzen in einer Oberfläche (10A) des Trägermaterials dergestalt, daß der Abstand der in einer ansonsten, im wesentlichen regelmäßigen Anordnung vorgesehenen Sacklöcher unter Ausbildung von Bereichsübergängen mit erhöhter Siliziumwanddicke bereichsweise geändert wird, wobei die Dicke der Siliziumwände zwischen den Bereichsübergängen um den Betrag des erhöhten Sacklöcherabstandes größer als die Dicke der Siliziumwände innerhalb der Bereiche ist;
(c) mindestens bereichsweises Anordnen einer Maskenschicht auf der Oberfläche (10A) und der Oberfläche der in Schritt (b) erzeugten Sacklöcher;
(d) Abtragen des Trägermaterials mindestens bis zum Boden der Sacklöcher unter Erhalten von Poren (11), welche sich von einer Oberfläche (10A) zur gegenüberliegenden Oberfläche (10B) des Trägermaterials durchgängig erstrecken;
(e) Entfernen der Maskenschicht;
(f) Unterwerfen des in Schritt (e) erhaltenen Trägermaterials einer thermischen Oxidation, so daß in Abhängigkeit von der Siliziumwanddicke die Bereiche mit dünneren Silizium-Wänden vollständig oxidiert werden, während bei den Bereichsübergängen mit erhöhter Wanddicke die Siliziumwände nicht vollständig oxidiert werden, so daß ein Siliziumkern in den Wänden bestehen bleibt.

Das in Schritt (a) bereitgestellte Trägermaterial aus Silizium kann beispielsweise n-dotiertes einkristallines Silizium (Si-Wafer) sein. Jedoch kann auch p-dotiertes einkristallines Silizium eingesetzt werden.

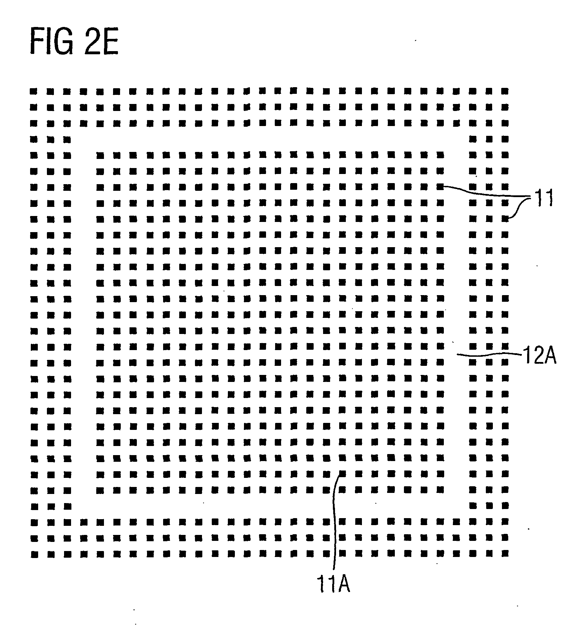

In Schritt (b) des Verfahrens erfolgt dann ein elektrochemischen Ätzen in das Silizium. Ein derartiges Verfahren ist beispielsweise aus EP 0 296 348, EP 0 645 621, WO 99/25026, DE 42 02 454, EP 0 553 465 oder DE 198 20 756 bekannt, auf die in vollem Umfang Bezug genommen wird und deren Offenbarungsgehalt insoweit Teil der vorliegenden Anmeldung sein soll. Im Rahmen eines derartigen elektrochemischen Ätzens können Sacklöcher bzw. Poren mit Aspektverhältnissen von beispielsweise 1 zu 300 und mehr in einer im wesentlichen regelmäßigen Anordnung in Silizium geätzt werden. Da das elektrochemische Porenätzverfahren bei geeignet gewählten Parametern eine Änderung des Porenabstandes (Pitch) in bestimmten Grenzen zulässt, kann durch Änderung des Porenabstandes und/oder das Weglassen einer ganzen Reihe von Poren in der ansonsten regelmäßigen Anordnung von Sacklöchern bzw. Poren die Dicke der resultierenden Silizium-Wände lokal verändert werden. Das Porenätzverfahren kann dabei zum einen unter Auslassung von Porenreihen bzw. einheiten und Beibehalten eines konstant gleichbleibenden Pitchabstandes durchgeführt werden. Zum anderen können auch zwei unterschiedliche Pitchabstände, einer für die jeweiligen Kompartimente, ein anderer für die entsprechenden Außenbereiche, vorgesehen werden; vgl. auch Fig. 2(E). Die vorgenannten Maßnahmen können in engen Grenzen variiert werden.

Um Poren zu erhalten, die durch das Trägermaterial bzw. Substrat (Si-Wafer) gehen und auf beiden Oberflächen (10A, 10B) geöffnet sind, wird in den Schritten (c), (d) und (e) nach der Ätzung der Sacklöcher auf der Rückseite des Si-Wafers z.B. durch eine KOH-Ätzung Silizium abgetragen, während die Vorderseite des Wafers und die Innenseite der Sacklöcher bzw. Poren durch eine Maskenschicht, wie z.B. eine durch CVD-Abscheidung erzeugte Siliziumnitridschicht in einer Dicke von beispielsweise 100 nm, geschützt werden. Die Maskenschicht kann dann in Schritt (e) beispielsweise mittels HF-Behandlung entfernt werden. Zum rückseitigen Abtragen des Si-Wafers eigenen sich gleichermaßen Sputter-, Laserablations und/oder Polierprozesse, beispielsweise ein CMP-Prozeß.

Dadurch wird ein Silizium-Wafer bzw. Silizium-Trägermaterial erzeugt, das matrixartig mit regelmäßigen Poren versehen ist, wobei die Poren durchgehende Röhren darstellen, welche die Vorder- und die Rückseite des Wafers miteinander verbinden.

Der Durchmesser dieser Poren kann nach deren Herstellung z.B. durch Ätzen in KOH vergrößert bzw. aufgeweitet werden. Wird Si(100) als Ausgangsmaterial verwendet, so entstehen bei einer solchen Ätzung auf Grund der Kristallstruktur im wesentlichen quadratische Poren. Geht man beispielsweise von Porendurchmesser von ca. 5 µm mit einem Abstand zwischen den Mittelpunkten zweier Poren (Pitch) von 12 µm aus, so kann dadurch der Porendurchmesser z.B. von 5 µm auf 10 bis 11 µm vergrößert werden. Die Dicke der Silizium-Wände zwischen den Poren nimmt dabei gleichzeitig auf 2 bis 1 µm ab. Auf diese Weise wird quasi ein quadratisches Gitter aus dünnen Silizium-Wänden erhalten. Die Tiefe der Poren bzw. die Länge der Silizium-Wände entspricht dabei der ursprünglichen Dicke des Silizium-Wafers, vermindert um die Dicke der beim Öffnen der Poren auf der Rückseite abgetragenen Si-Schicht.

In Schritt (f) wird das derart erhaltene Gitter in einem thermischen Oxidationsprozess z.B. bei einer Temperatur von 1050°C und einer Dauer von 18 Stunden durch Oxidation in Abhängigkeit von der jeweiligen Porenwanddicke in SiO₂ überführt. Die Struktur des Substrates wird dabei bis auf eine Volumenzunahme der Wandbereiche durch die Oxidation von Si zu SiO₂ im wesentlichen nicht verändert.

Wird in Schritt (b) der Abstand der Sacklöcher bzw. Poren voneinander periodisch, beispielsweise alle 5, 10 oder 20 Poren, geringfügig, z.B. um 1 µm, erhöht, so entsteht dadurch eine Überstruktur, die sich aus Bereichen bzw. Kompartments mit Arrays aus Poren (beispielsweise 5 x 5, 10 x 10, 20 x 20) zusammensetzt. Die Dicke der Siliziumwände ist zwischen diesen Bereichen um den Betrag des erhöhten Porenabstandes größer als die Dicke der Siliziumwände innerhalb der Bereiche. Bei anschließender Oxidation in Schritt (f) werden die Bereiche mit dünnen Silizium-Wänden vollständig zu SiO₂ oxidiert. Bei den Übergängen zwischen den Bereichen, die eine erhöhte Wanddicke aufweisen, werden die Siliziumwände jedoch nicht vollständig oxidiert, so daß ein Siliziumkern in den Wänden bestehen bleibt, wobei der Kern aus Silizium über den Querschnitt zur Außenseite der den Rahmen bildenden Wände hin jeweils in Siliziumdioxid übergeht. Es entstehen dadurch lokal vollständig transparente Bereiche aus SiO₂, die durch nicht-transparente Wände mit Siliziumkern vollständig voneinander getrennt sind.

Unmittelbar daran kann dann das Anbringen bzw. Binden von Linkermolekülen erfolgen. Solche Linkermoleküle unterliegen keiner spezifischen Beschränkung, solange sie befähigt sind, an die auf der Oberfläche der SiO₂-Schicht vorliegenden OH-Gruppen kovalent zu binden und weiter eine funktionelle Gruppe aufweisen, die zur kovalenten Bindung mit als Sonden in biologisch-chemischen Reaktionen einsetzbaren Fängermolekülen befähigt ist. Solche Linkermoleküle sind üblicherweise auf der Basis einer Silizium-organischen Verbindung. Derartige bifunktionelle Silizium-organische Verbindungen können beispielsweise Alkoxysilan-Verbindungen mit einer oder mehreren terminalen funktionalen Gruppen, ausgewählt aus Epoxy, Glycidyl, Chlor, Mercapto oder Amino, sein. Vorzugsweise ist die Alkoxysilan-Verbindung ein Glycidoxyalkylalkoxysilan, wie z.B. 3-Glycidoxypropyltrimethoxysilan, ein Mercaptoalkylalkoxysilan, wie z.B. γ-Mercaptopropyltrimethoxysilan, oder ein Aminoalkylalkoxysilan, wie z.B. N-β-(aminoethyl) γ-aminopropyltrimethoxysilan. Die Länge der als Spacer zwischen der funktionellen Gruppe, wie z.B. Epoxy bzw. Glycidoxy, welche mit dem Fängermolekül bzw. der Sonde bindet, und der Trialkoxysilangruppe wirkenden Alkylenreste unterliegt dabei keiner Beschränkung. Derartige Spacer können auch Polyethylenglykolreste sein.

Zur Endfertigung eines BioChips kann dann das Anbinden bzw. Koppeln von Fängermolekülen wie beispielsweise Oligonukleotiden bzw. DNA-Molekülen an das Trägermaterial über die Linkermoleküle nach den im Stand der Technik üblichen Verfahren erfolgen, beispielsweise mittels Behandeln des porösen Trägermaterials bei Verwendung von Epoxysilanen als Linkermoleküle durch anschließende Reaktion der terminalen Epoxidgruppen mit terminalen primären Aminogruppen oder Thiolgruppen von Oligonukleotiden bzw. DNA-Molekülen, die in entsprechenden Analyseverfahren als immobilisierte bzw. fixierte Fängermoleküle für die im zu untersuchenden Analyten vorliegenden Zielmoleküle fungieren. Dabei können beispielsweise die als Fängermoleküle verwendbaren Oligonukleotide unter Verwendung der Synthesestrategie, wie in Tet. Let. 22, 1981, Seiten 1859 bis 1862, beschrieben, hergestellt werden. Die Oligonukleotide können dabei während des Herstellungsverfahrens entweder an der 5-oder der 3-Endstellung mit terminalen Aminogruppen derivatisiert werden. Eine weitere Möglichkeit der Anbindung solcher Fängermoleküle an die Innenwandoberflächen der Poren kann durchgeführt werden, indem das Substrat zunächst mit einer Chlorquelle, wie Cl₂, SOCl₂, COCl₂ oder (COCl)₂, gegebenfalls unter Verwendung eines Radikalinitiators wie Peroxide, Azoverbindungen oder Bu₃SnH, behandelt wird und anschließend mit einer entsprechenden nucleophilen Verbindung, wie insbesondere mit Oligonukleotiden bzw. DNA-Molekülen, die terminale primäre Aminogruppen oder Thiolgruppen oder andere entsprechende funktionale Gruppen aufweisen, umgesetzt werden (siehe WO 00/33976).

Die erfindungsgemäße Vorrichtung kann dann die Funktion eines 96-Probenträgers mit der Dichte eines Mikroarrays aufweisen.

Weiterhin können auf Basis der erfindungsgemäßen Vorrichtung im Stand der Technik verfügbare Mikrochip-Technologien parallelisiert werden.

Die erfindungsgemäße Vorrichtung eignet sich insbesondere auch für die örtlich begrenzte, lichtgesteuerte Synthese von Molekülen an den Porenwänden. Ein weiterer Gegenstand der vorliegenden Erfindung betrifft somit ein Verfahren zum Steuern chemischer bzw. biochemischer Reaktionen bzw. Synthesen die Schritte:
- Bereitstellen einer erfindungsgemäßen Vorrichtung bzw. BioChips;
- Einleiten einer Synthesesubstanz in zumindest eine der Poren des Trägermaterials;
- Einkoppeln von Licht in die Pore zum optischen Anregen zumindest der Synthesesubstanz.

Für planare Substrate ist das Verfahren der lichtgesteuerten Synthese beispielsweise in EP 0 619 321 und EP 0 476 014 beschrieben. Auf den Offenbarungsgehalt dieser Druckschriften wird hinsichtlich des Aufbaus und des lichtgesteuerten Syntheseverfahren in vollem Umfang Bezug genommen, so daß diese Druckschriften insoweit zum Offenbarungsgehalt der vorliegenden Anmeldung gehören. Durch die effiziente Ausbreitung des Lichts in die Poren hinein können an den Porenwände photochemische Reaktionen getrieben bzw. gesteuert werden. Insbesondere können auf diese Weise komplexe sequentielle lichtgesteuerte chemische Reaktionen an den Porenbegrenzungsflächen ausgeführt werden.

Optisches Übersprechen zwischen den einzelnen Poren oder Bereichen/Kompartments wird durch die reflektierenden Wände aus im wesentlichen Silizium unterbunden. Damit wird ein Hauptproblem bei der lichtgesteuerten Synthese auf planaren Substraten gelöst.

Die vorliegende Erfindung wird nachfolgend unter Bezugnahme auf die Figuren beispielhaft beschrieben. Es zeigt:
- Fig. 1: eine schematische Ansicht einer in WO03/089925 vorgestellten Vorrichtung;
- Fig. 2: eine schematische Ansicht einer beispielhaften Ausführungsform einer erfindungsgemäßen Vorrichtung;
- Fig. 3: eine schematische Ansicht einer weiteren beispielhaften Ausführungsform einer erfindungsgemäßen Vorrichtung;
- Fig. 4: eine schematische Ansicht noch einer weiteren beispielhaften Ausführungsform einer erfindungsgemäßen Vorrichtung;

In Fig. 1 ist eine schematische Ansicht einer in WO03/089925 vorgestellten Vorrichtung gezeigt, die lokal transparente Bereiche aus SiO₂ aufweist, wobei diese transparenten Bereiche wiederum von einem reflektierenden Rahmen aus Wänden mit Siliziumkern umgeben sind.

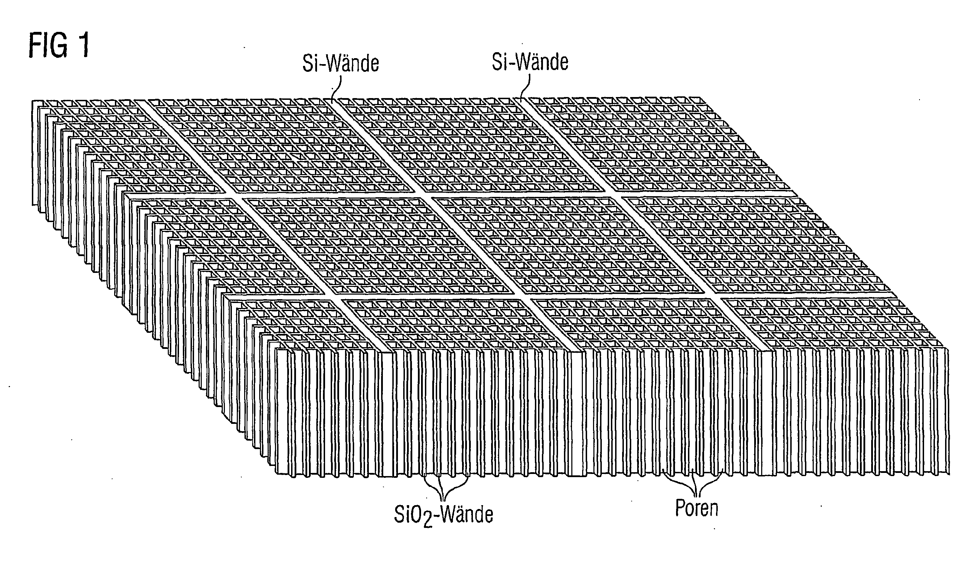

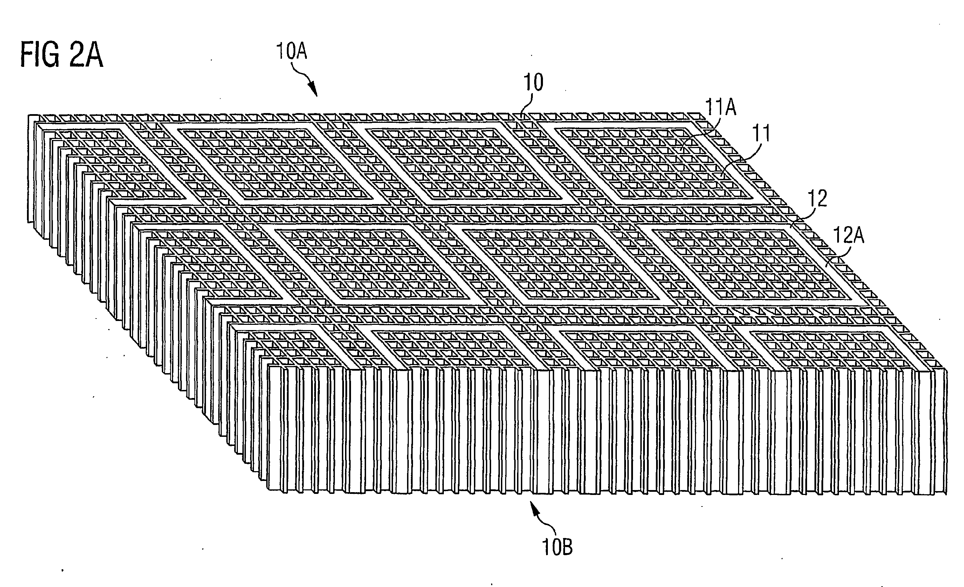

In Fig. 2(A) ist eine schematische Ansicht einer erfindungsgemäßen Vorrichtung (10) gezeigt, die lokal transparente Bereiche (11A) von Poren (11) mit Porenwänden aus SiO₂ aufweist, wobei jeder einzelne Rahmen (12) aus Wänden mit Siliziumkern (12A) innerhalb der Gesamtheit aller Rahmen aus Wänden mit einem Siliziumkern (12) von den ihn umgebenden Rahmen räumlich vollständig isoliert ist. Fig. 2(B) veranschaulicht die Definition bezüglich des Kompartiment-Aspektverhältnisses, wie vorstehend angegeben. Fig. 2(C) zeigt die Aufsicht und Fig. 2(D) die Seitenansicht eines Ausschnitts einer solchen Vorrichtung. Fig. 2(E) zeigt schematisch eine beispielhafte "Grundzelle" einer Struktur mit unterschiedlichem Pitchabstand innerhalb des Kompartments und im Außenbereich. Die beispielhafte Grundzelle umfasst 30 x 30 Poren mit einem 24 x 24 Porenarray (Pitch: 11,3 µm) innerhalb des Kompartments, wobei im Außenbereich ein Pitchabstand von 12,0 µm gegeben ist (Kompartmentwandstärke vor Oxidation: ~10 µm (Porendurchmesser : 10-11 µm) ; Kompartmentwandstärke nach Oxidation: ~5 µm).

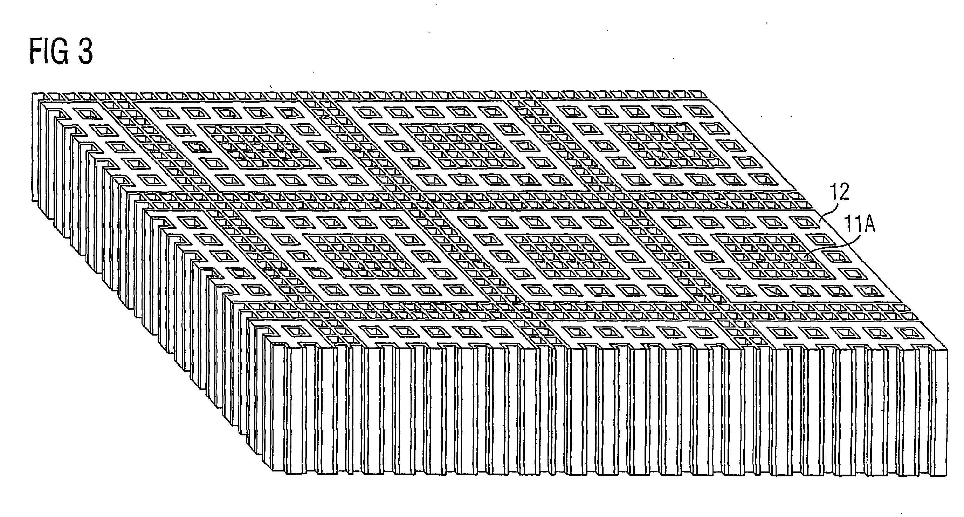

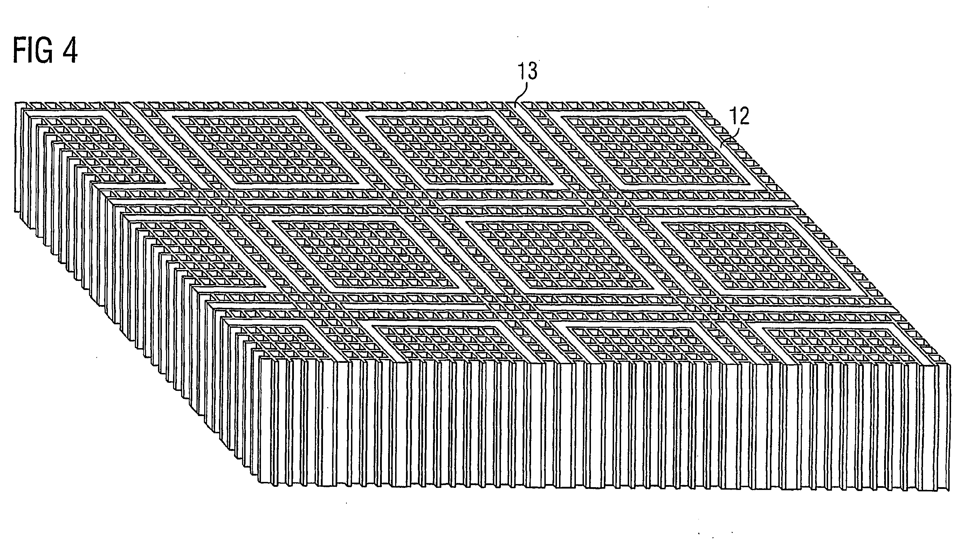

In Fig. 3 und Fig. 4 sind weitere Ausführungsformen der erfindungsgemäßen Vorrichtung gezeigt, die sich ebenfalls dadurch auszeichnen, daß jeder einzelne Rahmen (12) aus Wänden mit Siliziumkern (12A) innerhalb der Gesamtheit aller Rahmen aus Wänden mit einem Siliziumkern (12) von den ihn umgebenden Rahmen räumlich vollständig isoliert ist. In der Ausführungsform gemäß Fig. 3 ist der Rahmen aus Wänden mit Siliziumkern spezifisch gestaltet. In der Ausführungsform gemäß Fig. 4 sind zwischen den Rahmen aus Wänden mit Siliziumkern, die räumlich vollständig voneinander isoliert sind, wiederum Stege (13) aus Wänden mit einem Siliziumkern (12) vorgesehen, die räumlich von diesen Rahmen vollständig separiert sind.

In Fig. 5 ist eine weitere Ausführungsform der erfindungsgemäßen Vorrichtung gezeigt, die eine Hybridstruktur aus zusammenhängenden Kompartments und nichtzusammenhängenden Kompartments darstellt.

### Bezugszeichenliste

- 10: Trägermaterial auf Silizium-Basis
- 10A, 10B: Trägermaterialoberflächen
- 11: Pore
- 11A: Bereich, der mehrere Poren mit Porenwänden aus SiO₂ umfasst
- 12: Rahmen aus Wänden mit einem Siliziumkern
- 12A: Siliziumkern
- 13: Stege aus Wänden mit einem Siliziumkern

## Patentansprüche

1. Vorrichtung, umfassend ein flächig ausgebildetes makroporöses Trägermaterial (10) auf Silizium-Basis, das über mindestens einen Oberflächenbereich verteilt eine Vielzahl von Poren (11) mit einem Durchmesser im Bereich von 500 nm bis 100 µm aufweist, welche sich von einer Oberfläche (10A) zur gegenüberliegenden Oberfläche (10B) des Trägermaterials durchgängig erstrecken, wobei die Vorrichtung zwei oder mehrere Bereiche (11A) aufweist, die jeweils zwei oder mehrere Poren mit Porenwänden aus SiO₂ umfassen, und wobei diese Bereiche jeweils von einem zu den Längsachsen der Poren im wesentlichen parallel angeordneten, zu den Oberflächen (10A, 10B) hin offenen Rahmen bzw. Kasten (12) aus Wänden mit einem Siliziumkern (12A) umgeben ist, wobei der Kern aus Silizium über den Querschnitt zur Außenseite der den Rahmen bildenden Wände hin in Siliziumdioxid übergeht, und wobei jeder einzelne Rahmen (12) innerhalb der Gesamtheit aller Rahmen aus Wänden mit einem Siliziumkern (12A) vollständig von den ihn umgebenden Rahmen räumlich isoliert ist.

2. Vorrichtung nach Anspruch 1, wobei das Trägermaterial (10) eine Dicke zwischen 100 bis 1.000 µm, vorzugsweise 250 bis 450 µm aufweist.

3. Vorrichtung nach Anspruch 1 oder 2, wobei die Porendichte im Bereich von 10⁴ bis 10⁸/cm² liegt.

4. Vorrichtung nach einem der Ansprüche 1 bis 3, wobei die Poren (11) mit Porenwänden aus SiO₂ im wesentlichen quadratisch ausgebildet sind und die einzelnen Rahmen (12) aus Wänden mit einem Siliziumkern (12A) in im wesentlichen quadratischer oder rechteckiger Form vorliegen.

5. Vorrichtung nach einem der vorangegangenen Ansprüche, wobei an zumindest einer, innerhalb eines Rahmens (12) vorliegenden Pore (11) kovalent Fängermoleküle, ausgewählt aus der Gruppe, bestehend aus DNA, Proteinen und Liganden, gebunden sind.

6. Vorrichtung nach Anspruch 5, wobei die Fängermoleküle Oligonukleotidsonden sind.

7. Verwendung der Vorrichtung nach einem der Ansprüche 1 bis 6 als Basis für einen Probenträger in Verfahren zum Nachweis biochemischer Reaktionen und/oder Bindungen sowie hierfür insbesondere zur Untersuchung von enzymatischen Reaktionen, Nukleinsäure-Hybridisierungen, Protein-Protein-Wechselwirkungen und Protein-Liganden-Wechselwirkungen.

8. Verfahren zum Steuern chemischer bzw. biochemischer Reaktionen bzw. Synthesen, umfassend die Schritte:
- Bereitstellen einer Vorrichtung nach einem der vorhergehenden Ansprüche 1 bis 6;
- Einleiten einer Synthesesubstanz in zumindest eine der Poren des Trägermaterials;
- Einkoppeln von Licht in die Pore zum optischen Anregen zumindest der Synthesesubstanz.

## Claims

1. Device comprising a flatly designed macroporous support material (10) based on silicon, which has a multiplicity of pores (11) with a diameter in the range of from 500 nm to 100 µm distributed over at least one surface region and extending from one surface (10A) through to the opposite surface (10B) of the support material, wherein the device has two or more regions (11A) which comprise in each case two or more pores with SiO₂ pore walls, and wherein these regions are surrounded in each case by a frame or box (12) of walls with a silicon core (12A) which is arranged essentially parallel to the longitudinal axes of the pores and is open towards the surfaces (10A, 10B), wherein the silicon core merges into silicon dioxide over the cross section towards the outer side of the walls forming the frame, and wherein each individual frame (12) within the totality of all the frames of walls with a silicon core (12A) is completely spatially isolated from the frames surrounding it.

2. Device according to Claim 1, wherein the support material (10) has a thickness between 100 to 1000 µm, preferably 250 to 450 µm.

3. Device according to Claim 1 or 2, wherein the pore density is in the range of from 10⁴ to 10⁸/cm².

4. Device according to one of Claims 1 to 3, wherein the pores (11) with SiO₂ pore walls are designed essentially squarely and the individual frames (12) of walls with a silicon core (12A) are in an essentially square or rectangular shape.

5. Device according to one of the preceding claims, wherein capture molecules selected from the group consisting of DNA, proteins and ligands are covalently bound to at least one pore (11) located within a frame (12).

6. Device according to Claim 5, wherein the capture molecules are oligonucleotide probes.

7. Use of the device according to one of Claims 1 to 6 as a basis for a sample support in methods for detecting biochemical reactions and/or bindings and, in this context, in particular for the study of enzymatic reactions, nucleic acid hybridizations, protein-protein interactions and protein-ligand interactions.

8. Method for controlling chemical or biochemical reactions or syntheses, comprising the following steps:
- preparing a device according to one of the preceding Claims 1 to 6;
- introducing a synthesis substance into at least one of the pores of the support material;
- shining light into the pore in order to optically excite at least the synthesis substance.

## Revendications

1. Dispositif qui comprend un matériau de support macroporeux (10) de forme plate, à base de silicium, avec un grand nombre de pores (11) d'un diamètre compris dans la plage de 500 nm à 100 µm qui sont répartis sur au moins une partie de sa surface et qui s'étendent de manière continue depuis une surface (10A) jusqu'à la surface opposée (10B) du matériau de support, le dispositif présentant deux ou plusieurs parties (11A) qui comprennent chacune deux ou plusieurs pores dont les parois sont formées de SiO₂, ces parties étant toutes deux entourées par un encadrement ou caisson (12) constitué de parois à âme de silicium (12A) disposées essentiellement en parallèle à l'axe longitudinal des pores et ouvertes en direction des surfaces (10A, 10B), la section transversale de l'âme de silicium se prolongeant en dioxyde de silicium vers le côté extérieur des parois qui forment l'encadrement, chacun des encadrements (12) étant complètement isolé spatialement de celui des encadrements constitués de parois à âme de silicium (12A) qui l'entoure.

2. Dispositif selon la revendication 1, dans lequel le matériau de support (10) a une épaisseur comprise entre 100 et 1000 µm et de préférence entre 250 et 450 µm.

3. Dispositif selon les revendications 1 ou 2, dans lequel la densité en pores est comprise dans la plage de 10⁴ à 10⁸/cm².

4. Dispositif selon l'une des revendications 1 à 3, dans lequel les pores (11) dont les parois sont constituées de SiO₂ ont une forme essentiellement carrée, les différents encadrements (12) constitués de parois à âme de silicium (12A) présentant une forme essentiellement carrée ou rectangulaire.

5. Dispositif selon l'une des revendications précédentes, dans lequel des molécules de piégeage sélectionnées dans l'ensemble constitué de l'ADN, des protéines et des ligands sont liées par covalence à l'intérieur d'au moins un pore (11) présent à l'intérieur de l'encadrement (12).

6. Dispositif selon la revendication 5, dans lequel les molécules de piégeage sont des sondes d'oligonucléotides.

7. Utilisation du dispositif selon l'une des revendications 1 à 6 comme base pour un support d'échantillons dans un procédé de détection de réactions biochimiques et/ou de liaisons biochimiques, en particulier pour l'étude de réactions enzymatiques, d'hybridations d'acides nucléiques, d'interactions entre protéines et d'interactions entre protéines et ligands.

8. Procédé de contrôle de réactions ou de synthèses chimiques ou biochimiques, qui comprend les étapes qui consistent à :
- préparer un dispositif selon l'une des revendications 1 à 6 qui précèdent,
- introduire des substances de synthèse dans au moins l'un des pores du matériau de support et
- injecter de la lumière dans des pores pour exciter optiquement au moins la substance de synthèse.
